# EUROPÄISCHE PATENTSCHRIFT

(11) **EP 1 455 760 B1**
(45) Veröffentlichungstag und Bekanntmachung des Hinweises auf die Patenterteilung: **07.09.2005**
(21) Anmeldenummer: 02781328.6
(22) Anmeldetag: 25.11.2002
(51) Int. Cl.: A61K 9/28, A61K 9/20, A61K 31/70

(54) **ORALE FLUDARA REINST FORMULIERUNG MIT SCHNELLER FREISETZUNG DES WIRKSTOFFES**
ULTRAPURE ORAL FLUDARA FORMULATION WITH A FAST RELEASING ACTIVE SUBSTANCE
FORMULATION POUR ADMINISTRATION PAR VOIE ORALE, A LIBERATION RAPIDE DU PRINCIPE ACTIF, CONTENANT DU FLUDARA EXTRA-PUR

(30) Priorität: 20.12.2001 DE 10164510
(43) Veröffentlichungstag der Anmeldung: 15.09.2004
(73) Patentinhaber: Schering Aktiengesellschaft, 13342 Berlin (DE)
(72) Erfinder: HEIL, Wolfgang, 21435 Stelle (DE); TILSTAM, Ulf, B-1970 Wezmenbeek Oppem (BE); LIPP, Ralph, 14169 Berlin (DE); TACK, Johannes-Wilhelm, 13595 Berlin (DE)
(86) Internationale Anmeldenummer: PCT/EP2002/013252
(87) Internationale Veröffentlichungsnummer: WO 2003/053418

(56) Entgegenhaltungen:
- GB-A- 2 124 901
- US-B1- 6 174 873
- JAMES M. FORAN, DAVID OSCIER, JENNIFER ORCHARD: "Pharmacokinetic study of single doses of oral fludarabine phosphate in patients with "low-grade" non-hodgking's lymphoma" JOURNAL OF CLINICAL ONCOLOGY, Bd. 17, Nr. 5, Mai 1999 (1999-05), Seiten 1574-1579, XP008013976

## Beschreibung

Die vorliegende Erfindung betrifft eine schnellfreisetzende Tabletten-Formulierung mit >99,19 % reinem Fludara (reinst Fludara) 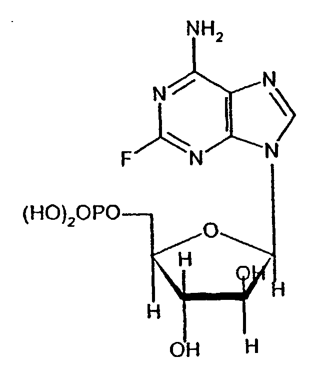 als Wirkstoff in definierter Zusammensetzung der Restverunreinigungen.

Tabletten-Formulierungen mit Fludara in einer Reinheit von < 98 % sind bereits bekannt In den nachfolgenden Arbeiten werden u. a. verschiedene Formulierungen und Dosierungen aufgeführt (7 Modern Pharmaceutics, Chapters 9 and 10, Banker & Rhodes, Editors, 1979; Liebennan et al., Pharmaceitical Dosage Forms: Tablets, 1981; Ansel, Introduction to Pharmaceutical Dosage Forms 2nd Edition, 1976).
In der US 3,903,297 wird exemplarisch eine Tabletten-Formulierungen aus Wirkstoff mit Lactose, mikrokristalliner Cellulose, kolloidalem Siliciumdioxid und Magnesiumstearat beschrieben (Beispiel 2). Daß eine Formulierung auch Croscaramellose-Na enthalten kann, ist ebenfalls exemplarisch beschrieben (Beispiel 5). In der WO 00/71134 werden allgemein Tabletten-Formulierungen aus Lactose, mikrokristalliner Cellulose, kolloidalem Siliciumdioxid, Croscaramellose-Na und Magnesiumstearat beschrieben. Ferner kann eine solche Zusammensetzung eine chemotherapeutische Substanz enthalten.

Aus der WO 97/40846 sind Tablettenüberzüge bekannt, die Hydroxypropylmethylcellulose, Titandioxid und Pigmente, wie z. B. Eisenoxidpigmente enthalten.

Aus der WO 00/50423 sind Tabletten-Formulierungen aus Lactose, mikrokristalliner Cellulose, Croscaramellose-Na, etc. bekannt, die sich schnell lösen.

In der US 6,197,785, EP 1065206, EP 819430, EP 1065204 und EP 985666 werden verwendbare Tabletten- Formulierungen für die orale Applikation beschrieben, die aus Lactose, mikrokristalliner Cellulose, kolloidalem Siliciumdioxid, Magnesiumstearat, Croscaramellose-Na, Talcum, etc. bestehen. Als Wirkstoff kann u. a. Fludara enthalten sein.

US 6,174,873 offenbart eine direkt verpresste magensaftresistent überzogene Tablette mit Fludarabine Monophosphate, PVP und Talkum.

Fludara-Formulierungen, die den Wirkstoff in einer Reinheit von >99,5 % aufweisen und eine Definierte Zusammensetzung der Verunreinigungen des Wirkstoffes in der Formulierung offenbaren, sind bisher nicht bekannt.

Aus der WO 99/29710 ist der Wirkstoff Fludara mit einer Reinheit von >99,19 % bekannt. Aber auch in diesem Stand der Technik wird keine definierte Zusammensetzung des reinen, in einer Formulierung enthaltenen Fludara gegeben.

Es wäre deshalb auch wünschenswert, eine stabile Tabletten-Formulierung zu haben, die Fludara in hoher Reinheit mit definierter Konzentration an Rest-Verunreinigungen aufweist, die schnellauflösend ist und damit den Wirkstoff schnell abgibt.

Es wurde nun gefunden, daß mit einer Tabletten-Formulierung, in der der Wirkstoff Fludara in einer Reinheit von >99,19 % in nicht-mikronisierter, aber gesiebter Form, mit definierter Konzentration an Rest-Verunreinigung vorliegt, die Nachteile der bekannten Tabletten überwindet.

Die Tablettenformulierung umfaßt den Wirkstoff in einer Menge von 5 bis 100 mg, vorzugsweise von 8 bis 75 mg, besonders bevorzugt in einer Menge von 10 bis 50 mg, ausgewählt in einer Menge von 10 bis 20 mg.

Die bevorzugten Formulierungsstoffe sind Lactose, kolloidales Siliciumdioxid mikrokristalline Cellulose (Avicel), Crosscaramellose-Natrium (Natriumcarboxymethylcellulose) und Magnesium-Stearat.

Es sind aber auch andere, dem Fachmann allgemein bekannte Formulierungsstoffe denkbar.

Die Formulierungsstoffe in der Tablette umfassen eine Gesamt-Menge von 100 bis 250 mg, vorzugsweise eine Gesamt-Menge von 120 bis 200 mg, besonders bevorzugt eine Gesamt-Menge von 130 bis 180 mg.

Gegenstand der vorliegenden Erfindung ist somit eine schnellfreisetzende Tabletten-Formulierung, umfassend 1 bis 100 mg des Wirkstoffes Fludara in einer Reinheit von >99,19 %, zusammen mit
Lactose Monohydrat,
kolloidalem Siliciumdioxid,
mikrokristalliner Cellulose (Avicel),
Croscaramellose-Na (Natriumcarboxymethylcellulose),
und Magnesiumstearat,
dadurch gekennzeichnet, daß die Verunreinigungen im Fludara einen Prozentsatz wie folgt nicht überschreiten:

| | |
|---|---|
| 0,02 % | 2-Fluor-9-(β-D-arabino-furanosyl)-9H-purin-6-amin, |
| 0,12 % | 6-Amino-9(5-O-phosphono-β-D- arabino-furanosyl)-9H-purin-2-ol, |
| 0,02 % | 2-Fluor-9H-purin-6-amin, |
| 0,02 % | 6-Amino-9H-purin-2-ol, |
| 0,05 % | 2-Fluor-9-(5-O-phosphono- β -D- ribofuranosyl)-9H-purin-6-amin, |
| 0,1 % | 9-(3,5-O-diphosphono- β -D- arabi-nofuranosyl)-2-fluoro-9H-purin-6-amin, |
| 0,1 % | 9-(2,5-O-diphosphono- β -D- ara-binofuranosyl)-2-fluoro-9H-purin-6-amin, |
| 0,02 % | 2-Fluor-9-(5-O-phosphono- β -D- arabinofuranosyl)-9H-purin-6-amin, |
| 0,06 % | 2-Ethoxy-9-(5-O-phosphono- β -D- arabinofuranosyl)-9H-purin-6-amin, |
| 0,02 % | 2-(6-Amino-9H-purin-2-yl)-9-(5-O- phosphono- β -D-ara-binofuranosyl)-9H-purin-6-amin und O,O'-Bis[2-(6-amino-2-fluor-9H- purin-9-yl)-5-deoxy-α-D-arabinofuranos-5-yl]-phosphat, |
| 0,1 % | 9-(2-Chlor-2-deoxy-5-phosphono- β -D-ara-binofuranosyl)-2-fluor-9H-purin-6-amin und |
| 0,1 % | 9-(2,5-Anhydro- β -D-arabino-furanosyl)-2- fluor-9H-purin-6-amin. |

Bevorzugt ist eine schnellfreisetzende Tabletten-Formulierung, die 1 bis 70,00 mg des Wirkstoffes Fludara in einer Reinheit von >99,19 %, zusammen mit
50 bis 100 mg Lactose Monohydrat,
0,1 bis 5 mg kolloidales Siliciumdioxid,
40 bis 100 mg mikrokristalline Cellulose (Avicel),
1 bis 10 mg Croscaramellose-Na (Natriumcarboxymethylcellulose) und
0,5 bis 10 mg Magnesiumstearat umfaßt.

Besonders bevorzugt sind solche schnellfreisetzenden Tabletten-Formulierungen, die 1 bis 50,00 mg des Wirkstoffes Fludara in einer Reinheit von >99,19 %, zusammen mit 60 bis 90 mg Lactose Monohydrat,
0,5 bis 1 mg kolloidales Siliciumdioxid,
50 bis 90 mg mikrokristalline Cellulose (Avicel),
1,5 bis 5 mg Croscaramellose-Na (Natriumcarboxymethylcellulose) und
1 bis 3 mg Magnesiumstearat umfassen.

Ausgewählt ist eine solche schnellfreisetzenden Tabletten-Formulierungen, die 10 mg des Wirkstoffes Fludara in einer Reinheit von >99,19 %, zusammen mit
74,75 mg Lactose Monohydrat,
0,75 mg kolloidales Siliciumdioxid,
60,00 mg mikrokristalline Cellulose (Avicel),
3,00 mg Croscaramellose-Na (Natriumcarboxymethylcellulose) und
1,5-2,00 mg Magnesiumstearat umfassen.

Bevorzugt sind auch solche Formulierungen, die den Wirkstoff Fludara in einer Reinheit von >99,37 % umfassen.

Noch bevorzugter sind solche Formulierungen, die den Wirkstoff Fludara in einer Reinheit von >99,57 % umfassen.

Besonders bevorzugt sind solche Formulierungen, die den Wirkstoff Fludara in einer Reinheit von >99,80 % umfassen.

Insbesondere bevorzugt sind solche Formulierungen, die den Wirkstoff Fludara in einer Reinheit von >99,85 % umfassen.

Die erfindungsgemäßen Formulierungen werden gemäß allgemein bekannter Methoden zu Preßmassen verarbeitet, die anschließend zu Tablettenkernen verpreßt werden. Diese Tablettenkerne können mit allgemein bekannten Methoden mit Überzügen versehen werden. Es können im Prinzip alle dem Fachmann bekannten Überzüge verwendet werden. Ein bevorzugter Überzug umfaßt z. B. die folgenden Bestandteile
1 bis 5 mg, vorzugsweise 1 bis 3 mg, besonders bevorzugt 2,250 mg Hydroxypropylmethylcellulose,
0,1 bis 1 mg, vorzugsweise 0,1 bis 0,8 mg, besonders bevorzugt 0,450 mg Talkum,
0,1 bis 5 mg, vorzugsweise 0,1 bis 2 mg, besonders bevorzugt 1,187 mg Titandioxid, ,
0,01 bis 0,1 mg, vorzugsweise 0,01 bis 0,05 mg, besonders bevorzugt 0,036 mg gelbes Eisenoxidpigment und
0,01 bis 0,1 mg, vorzugsweise 0,01 bis 0,05 mg, besonders bevorzugt 0,036 mg rotes Eisenoxidpigment.

Diese Überzüge sind ebenfalls Gegenstand der vorliegenden Erfindung.

Die erfindungsgemäßenTabletten-Formulierung können zur Herstellung eines Medikaments zur Behandlung von Krebs verwendet werden.

Die vorliegende Erfindung umfaßt somit auch die Verwendung der erfindungsgemäßen Formulierungen zur Herstellung eines Medikaments zur Behandlung von Krebs.

Die nachfolgenden Beispiele beschreiben die Herstellung der erfindungsgemäßen Fludara reinst Tabletten-Formulierungen sowie einen Vergleich der herkömmlichen Zusammensetzung des <98 %-igen reinen Fludara mit dem >99,19 % reinem Fludara.

### Beispiel 1

### Herstellung einer Tabletten-Formulierung

Für die Herstellung einer erfindungsgemäßen Tabletten-Formulierung wird der Wirkstoff Fludara (Fludarabinphosphat) zunächst gesiebt und anschließend mit Lactose Monohydrat, mikrokristalliner Cellulose (Avicel) und kolloidalem Siliciumdioxid zu einer etwa 30 %-igen Trockenmischung verarbeitet. Das Gemisch wird anschließend ebenfalls gesiebt. Die Qualität bzw. Partikelgröße wird mittels Siebanalyse geprüft. Anschließend werden Crosscaramellose Natrium (Natriumcarboxymethylcellulose) und Magnesiumstearat in weiteren Mischsequenzen sukzessive zur Trockenmischung zugegeben.
Die Preßmasse wird zu Tablettenkernen verpreßt.

Eine so hergestellte Tabletten-Formulierung umfaßt zum Beispiel die folgenden

| Einzelbestandteile | |
|---|---|
| Fludara >99,19 % reinst | 10,00 mg |
| Lactose Monohydrat | 74,75 mg |
| kolloidales Siliciumdioxid | 0,75 mg |
| mikrokristalline Cellulose (Avicel) | 60,00 mg |
| Croscaramellose-Na (Natriumcarboxymethylcellulose) | 3,00 mg |
| Magnesiumstearat | 1,5-2,00 mg |

Anschließend werden die Tablettenkerne mit einer wässrigen Filmsuspension lackiert. Ein solcher Filmüberzug umfaßt zum Beispiel die folgenden Bestandteile:

| | |
|---|---|
| Hydroxypropylmethylcellulose | 2,250 mg |
| Talkum | 0,450 mg |
| Titandioxid | 1,187 mg |
| Eisenoxidpigment, gelb | 0,036 mg |
| Eisenoxidpigment, rot | 0,036 mg |

Das Gesamtgewicht der Tablette beträgt 154 mg.

Die so hergestellten Filmtabletten könmnen anschließend weiter verarbeitet werden. So können die Filmtabletten z. B. in Alu-Blister verpackt werden, wodurch die Stabilität der Formulierung gewährleistet wird.

### Beispiel 2

Vergleich einer herkömmlichen Zusammensetzung des <98 %-igen (97,67%) reinen Fludara mit dem >99,19 %, bzw. >99,57 % reinen Fludara und dem über einen lonenaustauscher gereinigtem 99,19 %-igen Fludara.

Die Ergebnisse sind in der nachfolgenden Tabelle aufgeführt.

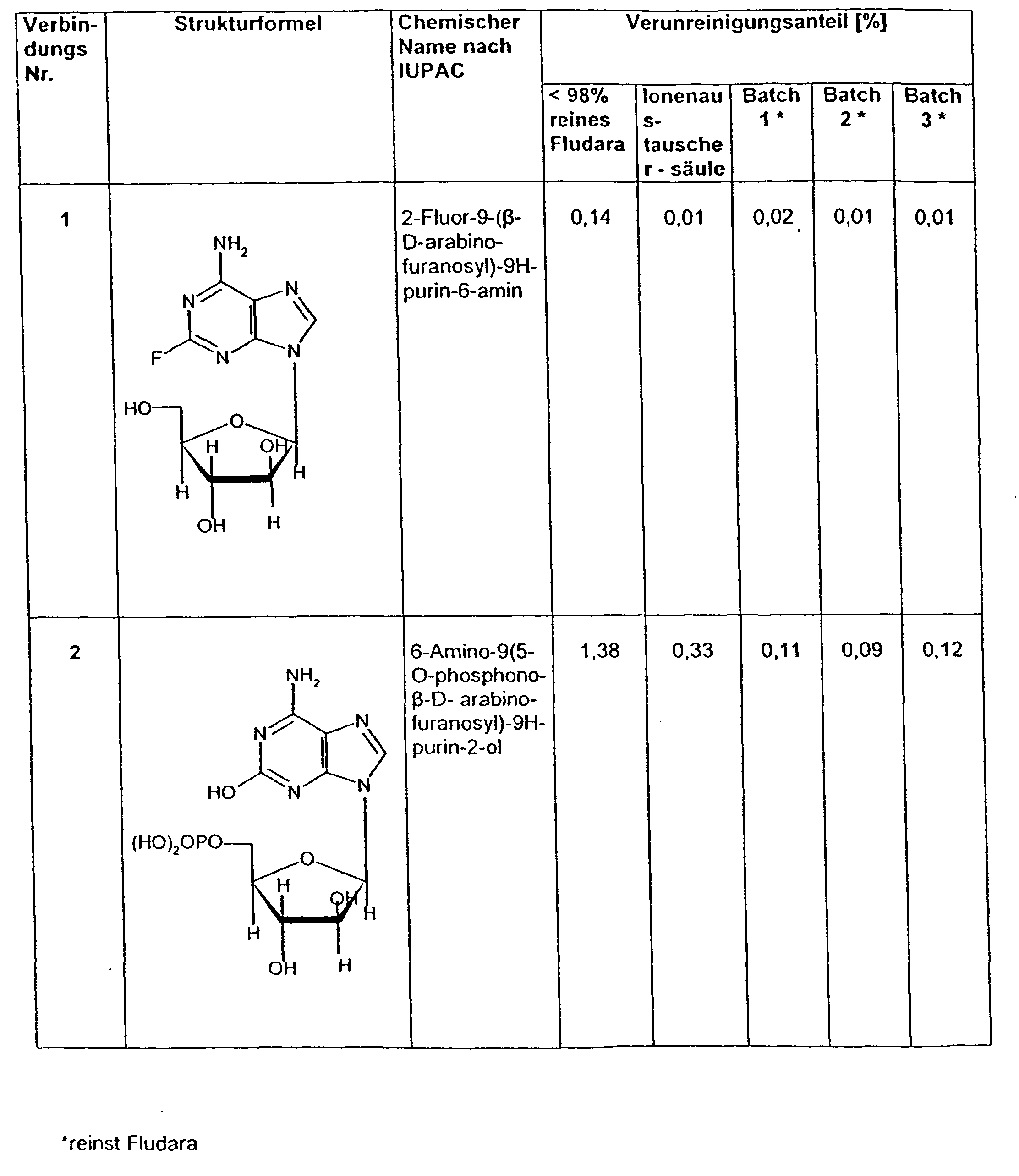

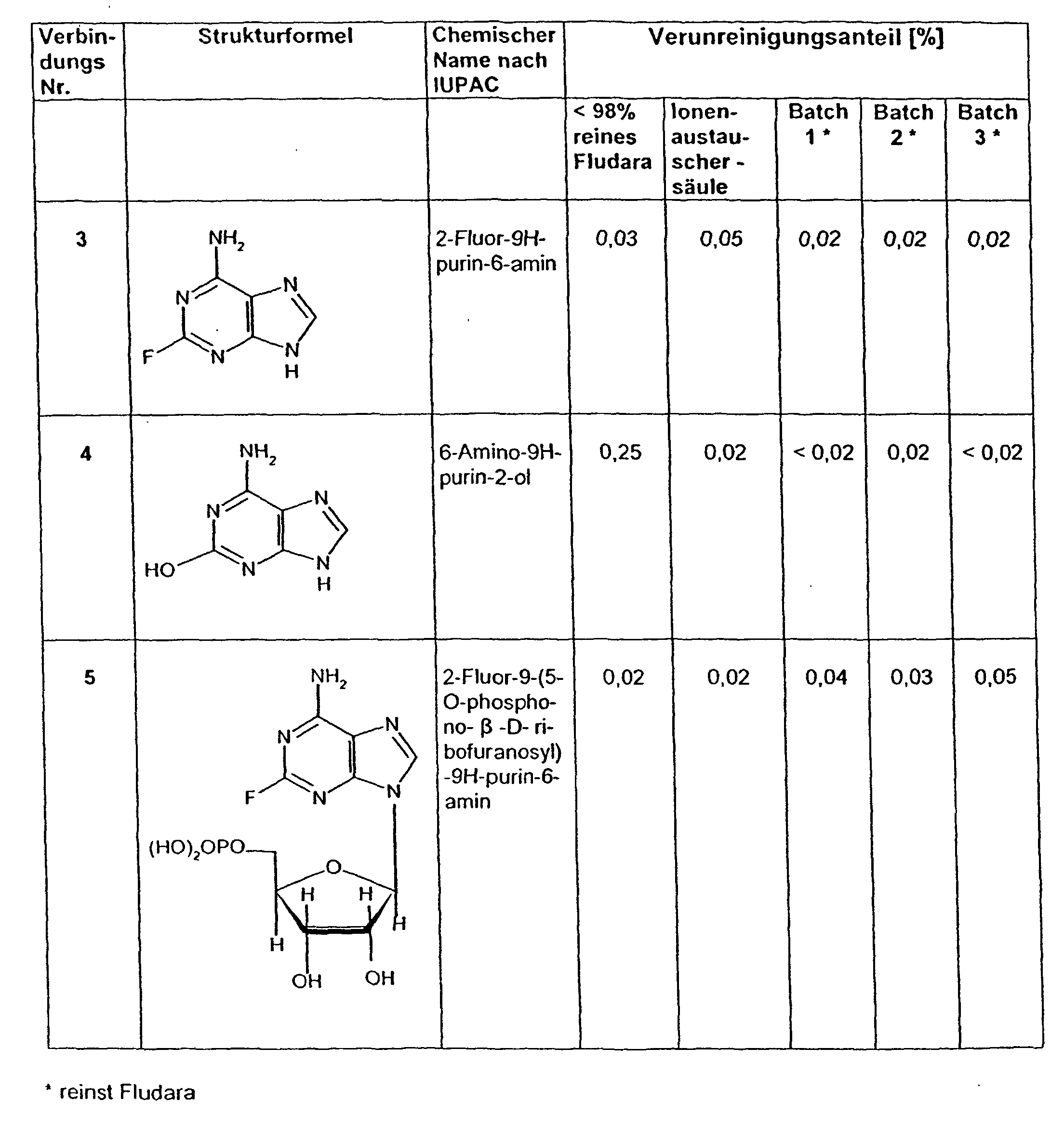

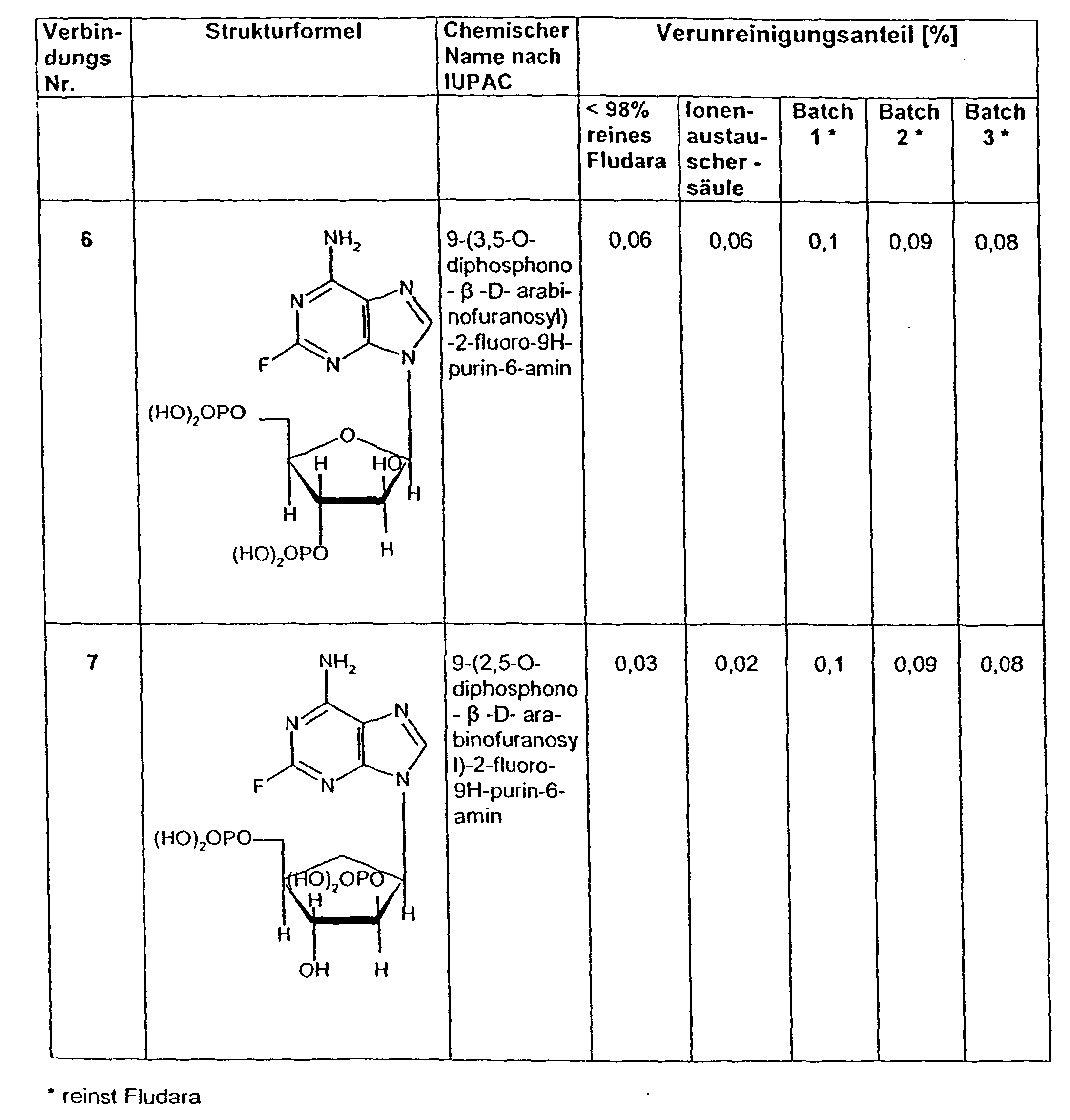

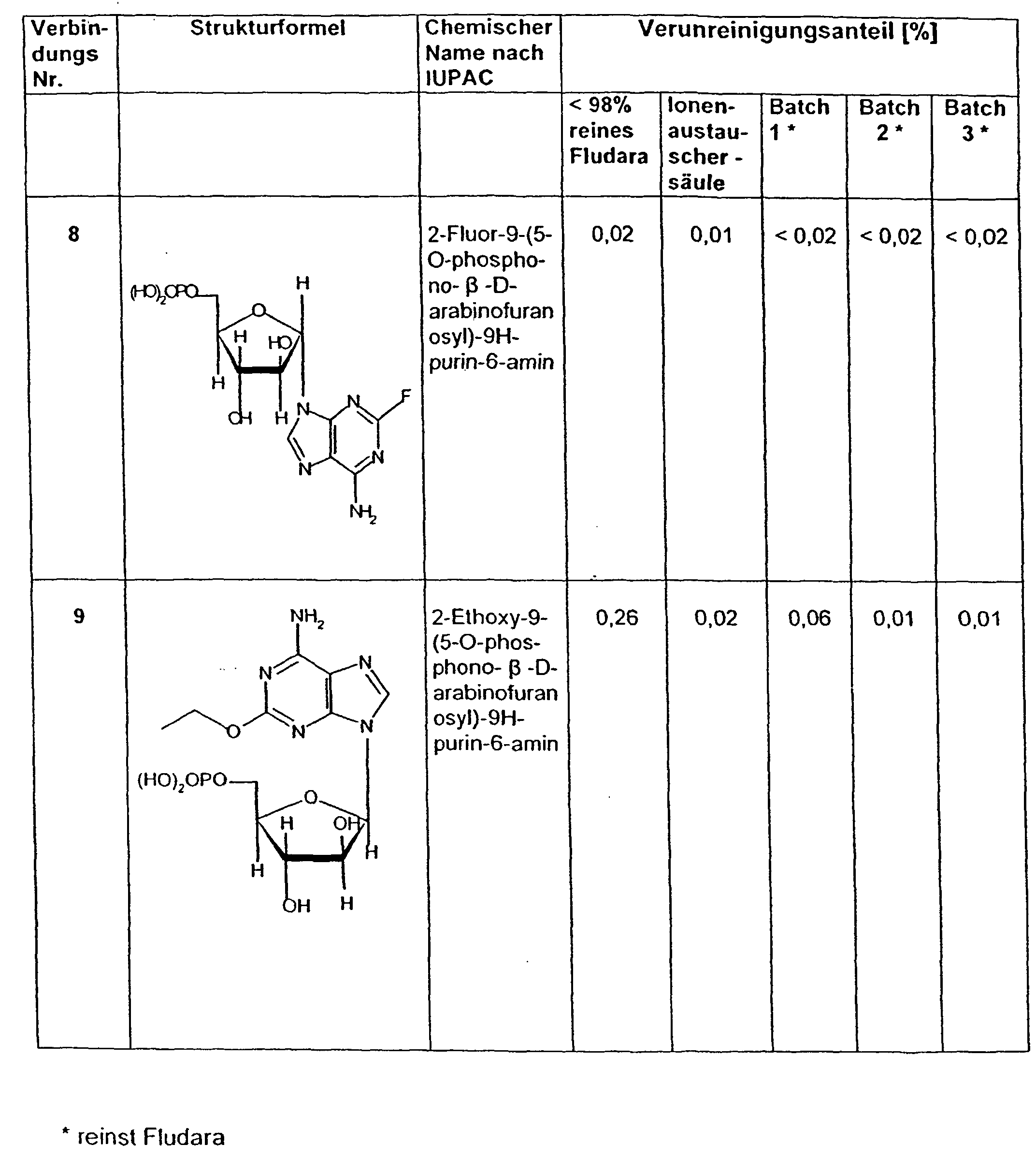

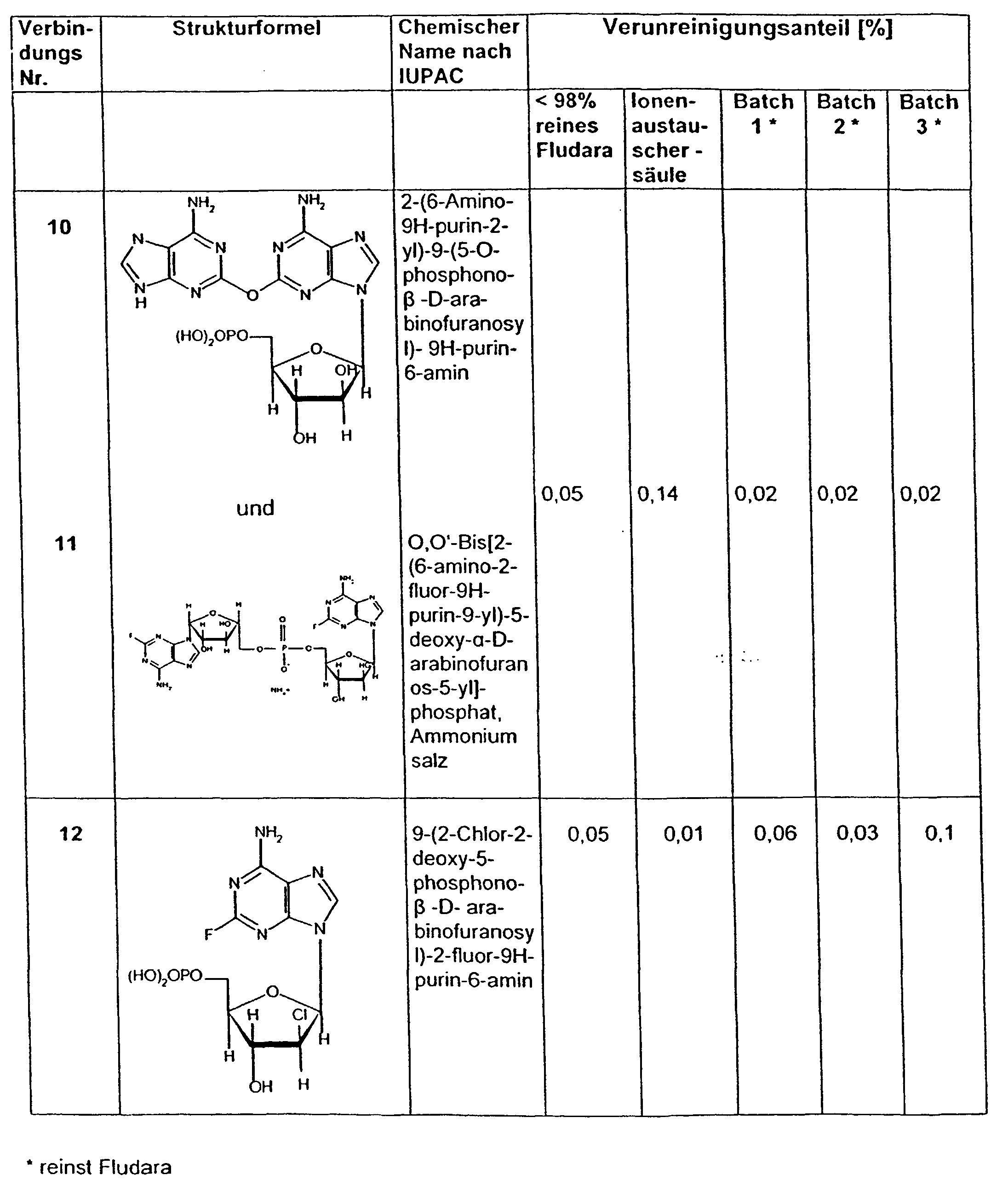

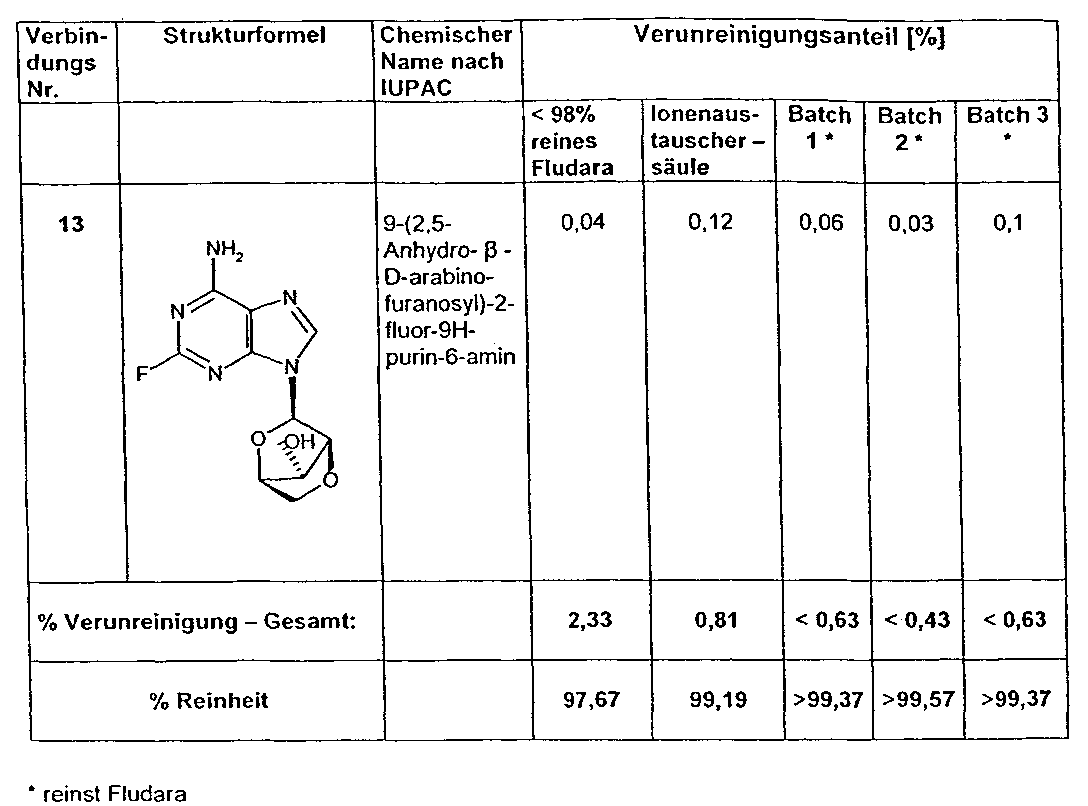

Die Ergebnisse zeigen, daß eine Formulierung aus käuflichem Fludara (maximal 97,67 %-iges Fludara) oder eine Formulierung mit über einen lonenaustauscher gereinigtem Fludara (maximal 99,19 %-iges Fludara) deutlich mehr verunreinigende Nebenprodukte aufweisen, als das in den erfindungsgemäßen Formulierungen enthaltene reinst Fludara (> 99,37 %-iges bis > 99,57 %-iges Fludara).

Mit herkömmlichen Reinigungsverfahren, wie der sehr potenten Ionenaustauschchromatographie läßt sich nur ein recht mäßiger Reinheitsgrad erzielen.
Die erfindungsgemäßen Fludara-Formulierungen enthalten das über das Natriumsalz freigesetzte reinst Fludara, was bereits in der WO 99/29710 beschrieben wird. Ein noch höherer Reinheitsgrad des Fludara kann über das Kaliumsalz (99,8 %) oder über das Lithiumsalz (99,85 %) hergestellt werden.

## Patentansprüche

1. Schnellfreisetzende Tabletten-Formulierung, umfassend 1 bis 100 mg des Wirkstoffes Fludara in einer Reinheit von >99,19 %, zusammen mit
Lactose Monohydrat,
kolloidalem Siliciumdioxid,
mikrokristalliner Cellulose (Avicel),
Croscaramellose-Na (Natriumcarboxymethylcellulose),
und Magnesiumstearat,
**dadurch gekennzeichnet, daß** die Verunreinigungen im Fludara einen
Prozentsatz wie folgt nicht überschreiten:
| | |
|---|---|
| 0,02 % | 2-Fluor-9-(β-D-arabino-furanosyl)-9H-purin-6-amin |
| 0,12 % | 6-Amino-9(5-O-phosphono-β-D- arabino-furanosyl)-9H-purin-2-ol |
| 0,02 % | 2-Fluor-9H-purin-6-amin |
| 0,02 % | 6-Amino-9H-purin-2-ol |
| 0,05 % | 2-Fluor-9-(5-O-phosphono- β -D- ribofuranosyl)-9H-purin-6-amin |
| 0,1 % | 9-(3,5-O-diphosphono- β -D- arabi-nofuranosyl)-2-fluoro-9H-purin-6-amin |
| 0,1 % | 9-(2,5-O-diphosphono- β -D- ara-binofuranosyl)-2-fluoro-9H-purin-6-amin |
| 0,02 % | 2-Fluor-9-(5-O-phospho-no- β -D- arabinofuranosyl)-9H-purin-6-amin |
| 0,06 % | 2-Ethoxy-9-(5-O-phos-phono- β -D- arabinofuranosyl)-9H-purin-6-amin |
| 0,02 % | 2-(6-Amino-9H-purin-2-yl)-9-(5-O- phosphono- β -D-arabinofuranosyl)- 9H-purin-6-amin und O,O'-Bis[2-(6-amino-2-fluor-9H- purin-9-yl)-5-deoxy-α-D-arabinofuranos-5-yl]-phosphat, |
| 0,1 % | 9-(2-Chlor-2-deoxy-5-phosphono- β -D- ara-binofuranosyl)-2-fluor-9H-purin-6-amin |
| 0,1 % | 9-(2,5-Anhydro- β -D-arabino-furanosyl)-2- fluoro-9H-purin-6-amine. |

2. Schnellfreisetzende Tabletten-Formulierung gemäß Anspruch 1, die 1 bis 70 mg des Wirkstoffes Fludara in einer Reinheit von >99,19 %, zusammen mit 50 bis 100 mg Lactose Monohydrat,
0,1 bis 5 mg kolloidales Siliciumdioxid,
40 bis 100 mg mikrokristalline Cellulose (Avicel),
1 bis 10 mg Croscaramellose-Na (Natriumcarboxymethylcellulose) und
0,5 bis 10 mg Magnesiumstearat umfaßt.

3. Schnellfreisetzende Tabletten-Formulierung gemäß den Ansprüchen 1 und 2, die 1 bis 50 mg des Wirkstoffes Fludara in einer Reinheit von >99,19 %, zusammen mit 60 bis 90 mg Lactose Monohydrat,
0,5 bis 1 mg kolloidales Siliciumdioxid,
50 bis 90 mg mikrokristalline Cellulose (Avicel),
1,5 bis 5 mg Croscaramellose-Na (Natriumcarboxymethylcellulose) und
1 bis 3 mg Magnesiumstearat umfaßt.

4. Schnellfreisetzende Tabletten-Formulierung gemäß den Ansprüchen 1 bis 3 , die 10 mg des Wirkstoffes Fludara in einer Reinheit von >99,19 %, zusammen mit 74,75 mg Lactose Monohydrat,
0,75 mg kolloidalem Siliciumdioxid,
60,00 mg mikrokristalline Cellulose (Avicel),
3,00 mg Croscaramellose-Na (Natriumcarboxymethylcellulose) und
1,5-2,00 mg Magnesiumstearat umfaßt.

5. Schnellfreisetzende Tabletten-Formulierung gemäß den Ansprüchen 1 bis 4 , die den Wirkstoff Fludara in einer Reinheit von >99,37 % umfaßt.

6. Schnellfreisetzende Tabletten-Formulierung gemäß den Ansprüchen 1 bis 5, die den Wirkstoff Fludara in einer Reinheit von >99,57 % umfaßt.

7. Schnellfreisetzende Tabletten-Formulierung gemäß den Ansprüchen 1 bis 6, die den Wirkstoff Fludara in einer Reinheit von >99,80 % umfaßt.

8. Schnellfreisetzende Tabletten-Formulierung gemäß den Ansprüchen 1 bis 7, die den Wirkstoff Fludara in einer Reinheit von >99,85 % umfaßt.

9. Schnellfreisetzende Tabletten-Formulierung gemäß den Ansprüchen 1 bis 8, **dadurch gekennzeichnet, daß** die Tablettenkerne von einem Überzug, der folgende Bestandteile umfaßt
1 bis 5 mg Hydroxypropylmethylcellulose,
0,1 bis 1 mg Talkum,
0,1 bis 5 mg Titandioxid,,
0,01 bis 0,1 mg gelbes Eisenoxidpigment und
0,01 bis 0,1 mg rotes Eisenoxidpigment, umgeben ist

10. Schnellfreisetzende Tabletten-Formulierung gemäß den Ansprüchen 1 bis 9, **dadurch gekennzeichnet, daß** die Tablettenkerne von einem Überzug, der folgende Bestandteile umfaßt
1 bis 3 mg Hydroxypropylmethylcellulose,
0,1 bis 0,8 mg Talkum,
0,1 bis 2 mg Titandioxid,
0,01 bis 0,05 mg gelbes Eisenoxidpigment und
0,01 bis 0,05 mg rotes Eisenoxidpigment, umgeben ist

11. Schnellfreisetzende Tabletten-Formulierung gemäß den Ansprüchen 1 bis 10, **dadurch gekennzeichnet, daß** die Tablettenkerne von einem Überzug, der folgende Bestandteile umfaßt
2,250 mg Hydroxypropyl-methylcellulose,
0,450 mg Talkum,
1,187 mg Titandioxid,
0,036 mg gelbes Eisenoxidpigment und
0,036 mg rotes Eisenoxidpigment, umgeben ist.

12. Verwendung der Tabletten-Formulierung gemäß den Ansprüchen 1 bis 11, zur Herstellung eines Medikaments zur Behandlung von Krebs.

## Claims

1. Fast-release tablet formulation comprising from 1 to 100 mg of the active ingredient Fludara in a purity of > 99.19%, together with
lactose monohydrate,
colloidal silicon dioxide,
microcrystalline cellulose (Avicel),
croscarmellose Na (sodium carboxymethylcellulose),
and magnesium stearate,
**characterized in that** the impurities in the Fludara do not exceed a percentage as follows:
| | |
|---|---|
| 0.02% | 2-fluoro-9-(β-D-arabinofuranosyl)-9H-purin-6-amine |
| 0.12% | 6-amino-9-(5-O-phosphono-β-D-arabinofuranosyl)-9H-purin-2-ol |
| 0.02% | 2-fluoro-9H-purin-6-amine |
| 0.02% | 6-amino-9H-purin-2-ol |
| 0.05% | 2-fluoro-9-(5-O-phosphono-β-D-ribofuranosyl)-9H-purin-6-amine |
| 0.1% | 9-(3,5-O-diphosphono-β-D-arabinofuranosyl)-2-fluoro-9H-purin-6-amine |
| 0.1% | 9-(2,5-O-diphosphono-β-D-arabinofuranosyl)-2-fluoro-9H-purin-6-amine |
| 0.02% | 2-fluoro-9-(5-O-phosphono-β-D-arabinofuranosyl)-9H-purin-6-amine |
| 0.06% | 2-ethoxy-9-(5-O-phosphono-β-D-arabinofuranosyl)-9H-purin-6-amine |
| 0.02% | 2-(6-amino-9H-purin-2-yl)-9-(5-O-phosphono-β-D-arabinofuranosyl)-9H-purin-6-amine and |
| | O,O'-bis[2-(6-amino-2-fluoro-9H-purin-9-yl)-5-deoxy-α-D-arabinofuranos-5-yl] phosphate |
| 0.1% | 9-(2-chloro-2-deoxy-5-phosphono-β-D-arabinofuranosyl)-2-fluoro-9H-purin-6-amine |
| 0.1% | 9-(2,5-anhydro-β-D-arabinofuranosyl)-2-fluoro-9H-purin-6-amine. |

2. Fast-release tablet formulation according to Claim 1, which comprises
from 1 to 70 mg of the active ingredient Fludara in a purity of > 99.19%, together with
from 50 to 100 mg of lactose monohydrate,
from 0.1 to 5 mg of colloidal silicon dioxide,
from 40 to 100 mg of microcrystalline cellulose (Avicel),
from 1 to 10 mg of croscarmellose Na (sodium carboxymethylcellulose) and
from 0.5 to 10 mg of magnesium stearate.

3. Fast-release tablet formulation according to Claims 1 and 2, which comprises
from 1 to 50 mg of the active ingredient Fludara in a purity of > 99.19%, together with
from 60 to 90 mg of lactose monohydrate,
from 0.5 to 1 mg of colloidal silicon dioxide,
from 50 to 90 mg of microcrystalline cellulose (Avicel),
from 1.5 to 5 mg of croscarmellose Na (sodium carboxymethylcellulose) and
from 1 to 3 mg of magnesium stearate.

4. Fast-release tablet formulation according to Claims 1 to 3, which comprises
10 mg of the active ingredient Fludara in a purity of > 99.19%, together with
74.75 mg of lactose monohydrate,
0.75 mg of colloidal silicon dioxide,
60.00 mg of microcrystalline cellulose (Avicel),
3.00 mg of croscarmellose Na (sodium carboxymethylcellulose) and
1.5-2.00 mg of magnesium stearate.

5. Fast-release tablet formulation according to Claims 1 to 4, which comprises the active ingredient Fludara in a purity of > 99.37%.

6. Fast-release tablet formulation according to Claims 1 to 5, which comprises the active ingredient Fludara in a purity of > 99.57%.

7. Fast-release tablet formulation according to Claims 1 to 6, which comprises the active ingredient Fludara in a purity of > 99.80%.

8. Fast-release tablet formulation according to Claims 1 to 7, which comprises the active ingredient Fludara in a purity of > 99.85%.

9. Fast-release tablet formulation according to Claims 1 to 8, **characterized in that** the tablet core is enveloped by a coating which comprises the following ingredients
from 1 to 5 mg of hydroxypropylmethylcellulose,
from 0.1 to 1 mg of talc,
from 0.1 to 5 mg of titanium dioxide,
from 0.01 to 0.1 mg of yellow iron oxide pigment and
from 0.01 to 0.1 mg of red iron oxide pigment.

10. Fast-release tablet formulation according to Claims 1 to 9, **characterized in that** the tablet core is enveloped by a coating which comprises the following ingredients
from 1 to 3 mg of hydroxypropylmethylcellulose,
from 0.1 to 0.8 mg of talc,
from 0.1 to 2 mg of titanium dioxide,
from 0.01 to 0.05 mg of yellow iron oxide pigment and
from 0.01 to 0.05 mg of red iron oxide pigment.

11. Fast-release tablet formulation according to Claims 1 to 10, **characterized in that** the tablet core is enveloped by a coating which comprises the following ingredients
2.250 mg of hydroxypropylmethylcellulose,
0.450 mg of talc,
1.187 mg of titanium dioxide,
0.036 mg of yellow iron oxide pigment and
0.036 mg of red iron oxide pigment.

12. Use of the tablet formulation according to Claims 1 to 11 for producing a medicament for the treatment of cancer.

## Revendications

1. Formulation en comprimé à libération rapide, comprenant de 1 à 100 mg du principe actif fludara en une pureté > 99,19 %, conjointement avec
du monohydrate de lactose,
du dioxyde de silicium colloïdal,
de la cellulose microcristalline (Avicel),
de la croscaramellose-Na (carboxyméthylcellulose sodique),
et du stéarate de magnésium,
**caractérisée en ce que** les impuretés dans le fludara ne dépassent pas un pourcentage comme suit :
| | |
|---|---|
| 0,02 % | de 2-fluoro-9-(β-D-arabino-furanosyl)-9H-purin-6-amine |
| 0,12 % | de 6-amino-9-(5-O-phosphono-β-D-arabinofuranosyl)-9H-purin-2-ol |
| 0,02 % | de 2-fluoro-9H-purin-6-amine |
| 0,02 % | de 6-amino-9H-purin-2-ol |
| 0,05 % | de 2-fluoro-9-(5-O-phosphono-β-D-ribofuranosyl)-9H-purin-6-amine |
| 0,1 % | de 9-(3,5-O-diphosphono-β-D-arabinofuranosyl)-2-fluoro-9H-purin-6-amine |
| 0,1 % | de 9-(2,5-O-diphosphono-β-D-arabinofuranosyl)-2-fluoro-9H-purin-6-amine |
| 0,02 % | de 2-fluoro-9-(5-O-phosphono-β-D-arabinofuranosyl)-9H-purin-6-amine |
| 0,06 % | de 2-éthoxy-9-(5-O-phosphono-β-D-arabinofuranosyl)-9H-purin-6-amine |
| 0,02 % | de 2-(6-amino-9H-purin-2-yl)-9-(5-O-phosphono-β-D-arabinofuranosyl)-9H-purin-6-amine et O,O'-bis[2-(6-amino-2-fluoro-9H-purin-9-yl)-5-désoxy-α-D-arabinofuranos-5-yl]phosphate |
| 0,1 % | de 9-(2-chloro-2-désoxy-5-phosphono-β-D-arabinofuranosyl)-2-fluoro-9H-purin-6-amine |
| 0,1 % | de 9-(2,5-anhydro-β-D-arabinofuranosyl)-2-fluoro-9H-purin-6-amine. |

2. Formulation en comprimé à libération rapide selon la revendication 1, comprenant
de 1 à 70 mg du principe actif fludara en une pureté > 99,19 %, conjointement avec de 50 à 100 mg de monohydrate de lactose,
de 0,1 à 5 mg de dioxyde de silicium colloïdal,
de 40 à 100 mg de cellulose microcristalline (Avicel),
de 1 à 10 mg de croscaramellose-Na (carboxyméthylcellulose sodique) et
de 0,5 à 10 mg de stéarate de magnésium.

3. Formulation en comprimé à libération rapide selon les revendications 1 et 2, comprenant
de 1 à 50 mg du principe actif fludara en une pureté > 99,19 %, conjointement avec de 60 à 90 mg de monohydrate de lactose,
de 0,5 à 1 mg de dioxyde de silicium colloïdal,
de 50 à 80 mg de cellulose microcristalline (Avicel),
de 1,5 à 5 mg de croscaramellose-Na (carboxyméthylcellulose sodique) et
de 1 à 3 mg de stéarate de magnésium.

4. Formulation en comprimé à libération rapide selon les revendications 1 à 3, comprenant
10 mg du principe actif fludara en une pureté > 99,19 %, conjointement avec 74,75 mg de monohydrate de lactose,
0,75 mg de dioxyde de silicium colloïdal,
60,00 mg de cellulose microcristalline (Avicel),
3,00 mg de croscaramellose-Na (carboxyméthylcellulose sodique) et
1,5 à 2,00 mg de stéarate de magnésium.

5. Formulation en comprimé à libération rapide selon les revendications 1 à 4, comprenant le principe actif fludara en une pureté > 99,37 %.

6. Formulation en comprimé à libération rapide selon les revendications 1 à 5, comprenant le principe actif fludara en une pureté > 99,57 %.

7. Formulation en comprimé à libération rapide selon les revendications 1 à 6, comprenant le principe actif fludara en une pureté > 99,80 %.

8. Formulation en comprimé à libération rapide selon les revendications 1 à 7, comprenant le principe actif fludara en une pureté > 99,85 %.

9. Formulation en comprimé à libération rapide selon les revendications 1 à 8, **caractérisée en ce que** le coeur de comprimé est entouré d'un enrobage comprenant les constituants suivants
de 1 à 5 mg d'hydroxypropylméthylcellulose,
de 0,1 à 1 mg de talc,
de 0,1 à 5 mg de dioxyde de titane,
de 0,01 à 0,1 mg de pigment oxyde de fer jaune et
de 0,01 à 0,1 mg de pigment oxyde de fer rouge.

10. Formulation en comprimé à libération rapide selon les revendications 1 à 9, **caractérisée en ce que** le coeur de comprimé est entouré d'un enrobage comprenant les constituants suivants
de 1 à 3 mg d'hydroxypropylméthylcellulose,
de 0,1 à 0,8 mg de talc,
de 0,1 à 2 mg de dioxyde de titane,
de 0,01 à 0,05 mg de pigment oxyde de fer jaune et
de 0,01 à 0,05 mg de pigment oxyde de fer rouge.

11. Formulation en comprimé à libération rapide selon les revendications 1 à 10, **caractérisée en ce que** le coeur de comprimé est entouré d'un enrobage comprenant les constituants suivants
2,250 mg d'hydroxypropylméthylcellulose,
0,450 mg de talc,
1,187 mg de dioxyde de titane,
0,036 mg de pigment oxyde de fer jaune et
0,036 mg de pigment oxyde de fer rouge.

12. Utilisation de la formulation en comprimé selon les revendications 1 à 11, pour la production d'un médicament destiné au traitement du cancer.
